(19) **Europäisches Patentamt**

**European Patent Office**

**Office européen des brevets**

(11) **EP 0 780 121 B1**

(12) **FASCICULE DE BREVET EUROPEEN**

(45) Date de publication et mention
de la délivrance du brevet:
**11.07.2001 Bulletin 2001/28**

(51) Int Cl.⁷: **A61K 9/08**, A61K 47/32

(21) Numéro de dépôt: **96402770.0**

(22) Date de dépôt: **17.12.1996**

(54) **Collyre destiné notamment au traitement de l'oeil sec**

Augentropfen insbesonders zur Behandlung des trockenen Auges

Eye lotion in particular for dry eye treatment

(84) Etats contractants désignés:
**AT BE CH DE DK ES FI FR GB GR IE IT LI LU MC
NL PT SE**

(30) Priorité: **19.12.1995 FR 9515061**

(43) Date de publication de la demande:
**25.06.1997 Bulletin 1997/26**

(73) Titulaire: **Laboratoire Chauvin S.A.
34009 Montpellier Cédex 1 (FR)**

(72) Inventeurs:
• **Maurin, Florence
34570 Vailhauques (FR)**
• **Latour, Elisabeth
34070 Montpellier (FR)**
• **Coquelet, Claude
34980 Saint Gely du Fesc (FR)**

(74) Mandataire: **Le Guen, Gérard et al
CABINET LAVOIX
2, place d'Estienne d'Orves
75441 Paris Cédex 09 (FR)**

(56) Documents cités:
**EP-A- 0 546 810          FR-A- 2 470 638
US-A- 4 744 980**

• **PHARM. ACT. HELV., vol. 67, no. 1, 1992, pages
5-10, XP000601398 UNLU N. ET AL: "A
Comparative Rheological Study on Carbopol
viscous Solutions and the Evaluation of their
Suitability as the Ophthalmic Vehicles and
artificial Tears"**
• **PROC. 1ST WORLD MEETING APGI / APV, vol.
9/11, Mai 1995, BUDAPEST, pages 16-17,
XP000603099 OECHSNER M. ET AL:
"Interactions between Carbopol 980 and
additionnal 2nd macromolecule in aqueous
formulations"**
• **SCI. PHARM. , vol. 49, no. 4, 1981, ALEXANDRIA,
pages 427-434, XP000646792 ELGINDY N.A. ET
AL: "Carbopol-Polyvinylpyrrolidone
Flocculation"**

Remarques:
Le dossier contient des informations techniques
présentées postérieurement au dépôt de la
demande et ne figurant pas dans le présent
fascicule.

## Description

La présente invention concerne un collyre destiné notamment au traitement de l'oeil sec.

Dans WO 84/04 680 et WO 84/04 681, on a déjà décrit des compositions destinées au traitement de l'oeil sec. Ces compositions sont constituées par des gels ou des solutions très visqueuses contenant des polymères polyanioniques tels que des produits vendus sous la marque Carbopol® qui sont des polymères de l'acide acrylique réticulés par de l'allyl saccharose ou de l'allylpentaérythritol.

C'est ainsi que WO 84/04 681 décrit par exemple un gel pouvant s'écouler qui comprend 0,2 % en poids de Carbopol 940. Ce gel a une viscosité de 12 400-12 600 mPa.s.

De tels gels sont toutefois d'un usage difficile et peuvent entraîner des sensations de collage des paupières.

On sait par ailleurs (document BF Goodrich Carbopol® - Résines solubles en milieu aqueux 1981) que l'addition de sels solubles tels que du chlorure de sodium ou du chlorure de calcium à des milieux contenant des Carbopol diminuent la viscosité de ces milieux.

On a d'ailleurs proposé dans WO 95/05 804 des compositions ophtalmiques contenant 0,1 à 10 % en poids de polymères polyanioniques, tels que les polymères de l'acide acrylique et notamment des Carbopol, et de 0,01 à 1,0 % en poids d'électrolytes fournissant des cations tels que $Na^+$, $Zn^{2+}$ ou $Al^{3+}$.

Ces compositions ont des viscosités plus faibles permettant une instillation.

La Demanderesse a toutefois constaté que de telles compositions sont opalescentes et peuvent donner des troubles visuels lors de l'instillation.

On a par ailleurs prétendu, dans une communication du Congrès de Budapest de l'APGI/APV, 9-11 mai 1995, que l'addition de polyvinylpyrrolidone diminuait la viscosité de formulations aqueuses à base de Carbopol 980 et que l'addition d'hydroxyde de sodium rendait ces formulations limpides.

Comme cela sera montré par la suite, ces résultats ne peuvent être reproduits.

La présente invention vise à fournir des compositions ophtalmiques destinées notamment au traitement de l'oeil sec qui présentent de faibles viscosités et qui soient limpides.

La présente invention a pour objet un collyre prêt à l'emploi destiné notamment au traitement de l'oeil sec comprenant en solution aqueuse

- un polymère gélifiant polyanionique,
- un tampon en une quantité suffisante pour réduire la viscosité du collyre à une valeur de 10 à 60 mPa.s, de préférence de 10 à 30 mPa.s, et
- une polyvinylpyrrolidone en une quantité suffisante pour que le collyre soit limpide.

La présente invention a également pour objet un procédé pour rendre limpide une composition opalescente contenant un polymère gélifiant polyanionique et un tampon en une quantité suffisante pour réduire la viscosité de la composition à une valeur de 10 à 60 mPa.s, consistant à ajouter une quantité suffisante de polyvinylpyrrolidone pour rendre limpide la composition.

Le polymère gélifiant polyanionique peut être notamment un polymère de l'acide acrylique réticulé et notamment un homopolymère de l'acide acrylique réticulé. Ces polymères sont notamment ceux vendus sous la marque Carbopol® et qui sont réticulés par de l'allyl saccharose ou de l'allylpentaérythritol tels que Carbopol 934, Carbopol 940 ou Carbopol 980. Ces polymères peuvent être présents notamment à des concentrations de 0,1 à 1 % en poids.

Le pH du collyre est avantageusement de 6,0 à 8,0 et notamment voisin de 7,0. Il peut être ajusté avec de l'hydroxyde de sodium.

Les tampons permettant de réduire la viscosité de collyre peuvent être des tampons tels le tampon phosphate potassique (pH 7,0), le tampon borate (pH 7,0), le tampon citrate (pH 7,0) et le tampon phosphate sodique (pH 7,0), les tampons phosphate sodique ou potassique étant préférés.

Les quantités de tampon à ajouter augmentent avec la concentration en polymères polyanioniques.

Les quantités de tampon à ajouter peuvent être aisément déterminées par des mesures de viscosité.

La viscosité indiquée est celle mesurée en utilisant un viscosimètre à mobile tournant HAAKE VT500 avec des cylindres coaxiaux NV comprenant un cylindre interne de rayons 17,85-20,1 mm et de hauteur 60 mm et un cylindre externe de rayons 17,5-20,5 mm et de largeur 0,35 mm. La viscosité indiquée est celle mesurée à 1200 $s^{-1}$ à une température de 22°C ± 1°C.

Les polyvinylpyrrolidones utilisées dans l'invention sont des polyvinylpyrrolidones solubles dans l'eau qui ont avantageusement des masses moléculaires moyennes en poids de 25000 à 100000, telles que la polyvidone PVP K30 (masse moléculaire moyenne en poids 45000).

Les quantités de polyvinylpyrrolidones à ajouter peuvent être aisément déterminées par des estimations de la limpidité. D'une manière générale, les quantités de polyvinylpyrrolidones à ajouter augmentent avec les quantités de polymère polyanionique. Pour des pourcentages de polymère polyanionique de l'ordre de 0,2 % en poids, des pourcentages de 1 % en poids de polyvinylpyrrolidone conviennent.

Un collyre préféré comprend, en solution aqueuse :

0,2 % de Carbopol 980,
1 % de polyvinylpyrrolidone K30,
du tampon phosphate sodique en quantité suffisante pour avoir une viscosité de 10 à 30 mPa.s.

Le collyre selon l'invention peut en outre contenir des adjuvants classiques des collyres tels que édétate de sodium qui joue le rôle de conservateur (et qui contribue partiellement à diminuer la viscosité), des conservateurs antimicrobiens tels que chlorure de benzalkonium, mercurothiolate sodique, ainsi que des isotonisants tels que sorbitol, mannitol.

On donnera ci-après des exemples de collyres selon l'invention qui à la fois ont une faible viscosité et sont limpides.

| EXEMPLE 1 | |
| --- | --- |
| Carbopol 934 | 0,200 g |
| Edétate de sodium | 0,050 g |
| Hydroxyde de sodium | 0,078 g |
| Sorbitol à 70 % cristallisable | 6,630 g |
| Polyvidone PVP K30 | 1,000 g |
| Dihydrogénophosphate de potassium | 0,074 g |
| Hydrogénophosphate de potassium | 0,200 g |
| Eau purifiée qsp | 100,000 g |
| Viscosité (à 1200 s$^{-1}$) | 11,4 mPa.s |

| EXEMPLE 2 | |
| --- | --- |
| Carbopol 980 | 0,500 g |
| Hydroxyde de sodium | 0,195 g |
| Polyvidone PVP K30 | 2,000 g |
| Dihydrogénophosphate de sodium, dihydraté | 0,540 g |
| Hydrogénophosphate de sodium, dodécahydraté | 2,260 g |
| Eau purifiée qsp | 100,000 g |
| Viscosité (à 1200 s$^{-1}$) | 30,0 mPa.s |

| EXEMPLE 3 | |
| --- | --- |
| Carbopol 980 | 0,750 g |
| Hydroxyde de sodium | 0,292 g |
| Polyvidone PVP K30 | 2,550 g |
| Dihydrogénophosphate de sodium, dihydraté | 1,250 g |
| Hydrogénophosphate de sodium, dodécahydraté | 5,200 g |
| Eau purifiée qsp | 100,000 g |
| Viscosité (à 1200 s$^{-1}$) | 27,7 mPa.s |

| EXEMPLE 4 | |
| --- | --- |
| Carbopol 980 | 0,200 g |
| Chlorure de benzalkonium Solution à usage ophtalmique | 0,010 g |
| Edétate de sodium | 0,050 g |
| Polyvidone PVP K30 | 1,000 g |
| Hydroxyde de sodium | 0,078 g |

(suite)

| EXEMPLE 4 | |
|---|---|
| Dihydrogénophosphate de sodium, dihydraté | 0,052 g |
| Hydrogénophosphate de sodium, dodécahydraté | 0,217 g |
| sorbitol à 70 % cristallisable | 6,360 g |
| Eau purifiée qsp | 100,000 g |
| Viscosité (à 1200 s$^{-1}$) | 15,0 mPa.s |

On donnera ci-après les résultats d'une étude sur le lapin sur la remanence oculaire de collyres selon l'invention comparée à celle d'un gel contenant la même concentration de Carbopol (0,2 % en poids).

La rémanence à la surface oculaire des compositions a été déterminée en étudiant la cinétique de l'augmentation du volume lacrymal artificiellement induite, comparativement à l'effet d'une solution ophtalmique ne contenant pas de polymères.

Les expériences ont été réalisées sur des lapins mâles albinos néo-zélandais pesant de 2,3 kg à 3,6 kg, provenant de l'élevage Charles River France (St Aubin les Elbeuf 76410 CLEON) acclimatés pendant 5 jours minimum dans l'animalerie (température 19±2°C ; humidité relative : 55±10 % ; éclairage : 12 heures d'éclairage artificiel - 12 heures de nuit).

Les collyres testés avaient la composition suivante :

| | A | B | C |
|---|---|---|---|
| Carbopol 980 | 0,20 g | 0,20 g | - |
| Edétate de sodium | 0,05 g | 0,05 g | 0,05 g |
| Sorbitol (70 %) cristallisable | 6,82 g | 6,36 g | 6,36 g |
| Chlorure de benzalkonium | 0,01 g | 0,01 g | 0,01 g |
| Dihydrogénophosphate de sodium dihydraté | 0,018 g | 0,052 g | 0,052 g |
| Hydrogénophosphate de sodium dodécahydraté | 0,077 g | 0,217 g | 0,217 g |
| Polyvidone PVP K30 | x1,00 g | 1,00 g | 1,00 g |
| Hydroxyde de sodium | 0,078 g | 0,078 g | qs pH7 |
| Eau purifiée qsp | 100,0 g | 100,0 g | 100,0 g |
| pH | 7 | | |

Le volume lacrymal a été mesuré selon une technique de dilution d'une solution de fluorescéine, proche des méthodes utilisées par Mishima et al. (Invest. Ophthalmol.,5(3), 264-276, 1966) et Göbbels et al. (Graefe's Arch. Clin. Exp. Ophthalmol., 229, 147-149, 1991) chez l'homme. Le principe général de cette méthode est le suivant : une solution de fluorescéine de concentration connue est instillée sous un volume le plus faible possible afin d'éviter le larmoiement réflexe puis immédiatement répartie de façon homogène dans le film lacrymal par clignements successifs. Un prélèvement de larmes est effectué tout de suite après ; la fluorescence de cet échantillon est déterminée par fluorimétrie. Le volume lacrymal est calculé selon l'équation :

$$V = \frac{Vs.Qi}{Qs} - Vi$$

V : volume lacrymal
Vs : volume du prélèvement de larmes
Qi : quantité de fluorescéine instillée
Qs : quantité de fluorescéine dans le prélèvement de larmes
Vi : volume de la solution de fluorescéine instillée

Les animaux ont été anesthésiés par administration intramusculaire d'un mélange kétamine (35 mg/kg) - xylazine (5 mg/kg) afin de faciliter le prélèvement de larmes et de rendre la mesure du volume lacrymal plus fiable ; en effet, chez l'animal vigile, des clignements importants sont observés à l'approche du microcapillaire.

Dix minutes après injection de l'anesthésique, 1 µl d'une solution aqueuse de fluorescéine à 1 % a été instillé dans le cul-de-sac conjonctival inférieur à l'aide d'un microcapillaire. Les paupières ont été fermées manuellement deux fois en évitant toute pression sur le globe oculaire. Cinq secondes après l'instillation de fluorescéine, 1 µl de larmes a été prélevé par capillarité au niveau du ménisque inférieur en évitant tout contact du microcapillaire avec la paupière inférieure et la surface oculaire.

Le prélèvement de larmes a été dilué dans 4 ml de tampon phosphate isotonique pH7,4 ($NaH_2PO_4$ : 32 mM; $Ma_2HPO_4$ : 104 mM). La fluorescence de ces échantillons a été mesurée à température ambiante au spectrofluorimètre (longueur d'onde d'excitation : 496 nm ; longueur d'onde d'émission : 506 nm) par rapport à celle d'une solution de référence contenant 1,6 µg de fluorescéine dans 4 ml de tampon phosphate (100 % de fluorescence). Le 0 a été réglé avec le tampon phosphate seul.

La quantité de fluorescéine contenue dans le prélèvement de larmes (Qs) a été calculée à partir du pourcentage de fluorescence relatif de l'échantillon (x) selon la formule :

$$Qs\ (\mu g) = \frac{x(\%)\ x\ 1,6\ \mu g}{100}$$

Le volume lacrymal exprimé en µl a été calculé selon l'équation donnée précédemment dans laquelle :

Vi = 1 µl      Qi = 10 µg      Vs = 1 µl

Des contrôles préalables ont permis de vérifier que la méthode de dosage était linéaire dans la gamme de concentrations des échantillons testés dans cette étude et que l'addition de larmes ou des formules testées ne modifiait pas l'intensité de fluorescence de solutions de fluorescéine de référence.

Les animaux ont été traités dans un seul oeil par une instillation de 25 µl de la préparation testée dans le cul-de-sac conjonctival inférieur, à différents temps avant la mesure du volume lacrymal. Les paupières ont ensuite été fermées manuellement une fois.

Pour chaque formule testée et chaque temps, un lot d'animaux recevant dans les mêmes conditions 25 µl d'une solution isotonique de sorbitol a été inclus comme contrôle (sorbitol 70 % à 7,5 % p/p).

Lorsque des traitements différents (formulations ou temps de prétraitement différents) ont été testés chez les mêmes animaux, un intervalle de 3 jours minimum a été respecté entre les expériences.

Le volume lacrymal est exprimé sous forme de moyenne ± écart-type.

Dans de très rares cas, une anesthésie insuffisante ou une mauvaise homogénéisation de la solution de fluorescéine après fermeture manuelle des paupières ont été observées. Les mesures correspondantes n'ont pas été prises en compte dans le calcul des moyennes.

Les valeurs moyennes obtenues dans chaque lot (contrôle et traité) ont été comparées à l'aide du test U de Mann et Whitney.

L'analyse statistique a été réalisée à l'aide du logiciel Statworks. La différence est considérée comme significative lorsque p est inférieure ou égale à 0,05.

Les collyres A et B sont des collyres selon l'invention tandis que le collyre C ne contient pas de Carbopol.

La composition du gel de comparaison était la suivante :

| | |
|---|---|
| Carbopol 940 | 0,2000 g |
| Chlorure de benzalkonium | 0,0100 g |
| Hydroxyde de sodium | 0,0660 g |
| Sorbitol à 70 % cristallisable | 5,7143 g |
| Eau purifiée qsp | 100,0000 g |

Les viscosités des compositions ont été mesurées avec un appareil CARRI-MELD CSL 100

- Mobile : plan/cône inox pour le gel de diamètre 4 cm, angle : 2°, entrefer = 56

plan/cône pour les autres compositions de diamètre 6 cm, angle : 2° et entrefer 67.

Le gradient de vitesse est de 0 à 1 200 $s^{-1}$ en 3 minutes à une température de 34,0 ± 0,1°C.

| Composition | Viscosité apparente* (mPa.s) |
|---|---|
| A | 30,4 ± 0,3** |
| B | 15,0 ± 0,1 |
| Gel | 256,5 ± 3,4 |

* Vitesse de cisaillement : 1 200 $s^{-1}$

** Moyenne ± écart-type (3 mesures)

Les résultats obtenus avec le gel sont les suivants :

| Temps (mn) | Volume lacrymal (µl) | |
|---|---|---|
| | Contrôle | Gel |
| 10 | 4,82 ± 0,23 (4) | 13,45 ± 5,28** (11) |
| 20 | 4,59 ± 0,96 (4) | 9,69 ± 2,62** (12) |
| 40 | 3,77 ± 0,26 (4) | 7,47 ± 2,28** (11) |
| 60 | 4,09 ± 0,78 (4) | 5,92 ± 1,43* (12) |
| 120 | 4,46 ± 0,37 (4) | 5,03 ± 1,06 (12) |

*$p < 0,05$;
** $p < 0,01$ comparé au lot contrôle
nombre d'animaux entre parenthèses

Les résultats obtenus avec le collyre A selon l'invention, sont les suivants :

| Temps (mn) | Volume lacrymal (µl) | |
|---|---|---|
| | Contrôle | Collyre A |
| 10 | 4,16 ± 0,52 (5) | 13,59 + 6,22** (11) |
| 20 | 4,07 ± 0,68 (5) | 10,04 ± 2,06** (11) |
| 40 | 4,64 ± 0,56 (5) | 7,52 ± 1,90** (11) |
| 60 | 4,25 ± 0,38 (5) | 4,92 ± 0,82 (8) |
| 120 | 5,16 ± 0,78 (5) | 5,18 ± 0,60 (10) |

** $p < 0,01$ comparé au lot contrôle
nombre d'animaux entre parenthèses

Les résultats obtenus avec le collyre B (selon l'invention) sont les suivants :

| Temps (mn) | Volume lacrymal (µl) | |
|---|---|---|
| | Contrôle | Collyre B |
| 10 | 4,56 ± 0,84 (5) | 12,42 ± 3,40** (11) |
| 20 | 4,72 ± 0,64 (4) | 10,51 ± 2,46** (11) |
| 40 | 4,84 ± 0,75 (5) | 7,80 ± 1,91** (11) |
| 60 | 4,33 ± 1,06 (5) | 5,54 ± 1,63 (11) |
| 120 | 4,98 ± 1,12 (5) | 5,28 ± 1,03 (7) |

** $p < 0,01$ comparé au lot contrôle
nombre d'animaux entre parenthèses

Les résultats obtenus avec le collyre C (exemple comparatif) sont les suivants :

| Temps (mn) | Volume lacrymal (µl) | |
|---|---|---|
| | Contrôle | Collyre C |
| 5 | 8,08 ± 1,75 (6) | 12,51 ± 3,16** (10) |
| 10 | 5,85 ± 1,36 (6) | 7,12 ± 2,53 (9) |
| 20 | 5,30 ± 1,22 (6) | 6,02 ± 1,47 (10) |

** $p < 0,01$ comparé au lot contrôle
nombre d'animaux entre parenthèses

Ces résultats mettent en évidence une remanence oculaire avec les collyres A et B selon l'invention pratiquement comparable à celle du gel. En revanche, le collyre C qui contient de la polyvinylpyrrolidone mais qui ne contient pas de Carbopol n'a qu'un effet de durée très limitée.

On donnera par ailleurs ci-après des résultats destinés à montrer l'influence de l'addition de polyvinylpyrrolidone et des tampons à des compositions aqueuses à base de Carbopol sur la viscosité et la limpidité. Ces résultats sont rassemblés dans le tableau suivant :

|  | formule 1 | formule 2 | formule 3 | formule 4 | formule 5 |
|---|---|---|---|---|---|
| Carbopol 980 | 0,2 g | id. | id. | id. | id. |
| EDTA | 0,05 g | id. | id. | id. | id. |
| Soude | qs pH=7 | id. | id. | id. | id. |
| Sorbitol | qs isotonie | id. | id. | id. | id. |
| PVP K 30 | - | 1 g | 6 g | - | 1 g |
| $NaH_2PO_4, 2H_2O$ | - | - | - | 0,052 g | 0,052 g |
| $NaH_2PO_4, 12H_2O$ | - | - | - | 0,217 g | 0,217 g |
| eau | | | | | |
| aspect | qsp 100 ml | id. | id. | id. | id. |
| viscosité en mPa.s | limpide | limpide | opalescent | opalescent | limpide |
| (à 1200 $s^{-1}$) | 100 | 130 | 95 | 16 | 19 |

Ces résultats montrent que, contrairement à ce qui a été annoncé dans la communication du Congrès de Budapest mentionnée, ci-dessus, l'addition de polyvinylpyrrolidone ne modifie pas de façon notable la viscosité de compositions à base de Carbopol aussi bien à des concentrations de 1 % que de 6 % et que, d'autre part, l'addition de 6 % de polyvinylpyrrolidone induit une opalescence (qui ne peut être masquée par l'addition de soude puisque l'on ne peut dépasser la neutralité) . Seule l'addition de tampon permet de diminuer la viscosité (formules 4 et 5) et l'opalescence est supprimée par addition de polyvinylpyrrolidone (formule 5). L'addition d'une polyvinylpyrrolidone K25 telle qu'enseignée dans la communication à la place de la polyvinylpyrrolidone K30 donne les mêmes résultats.

On donnera enfin des résultats montrant qu'il est facile de déterminer la quantité de tampon à ajouter pour avoir la viscosité souhaitée.

A une composition identique à celle de l'exemple 4, mais sans tampon et sans conservateur, on a ajouté des quantités croissantes de tampon phosphate sodique de composition :

| $NaH_2PO_4, 2H_2O$ | 0,87 g |
|---|---|
| $NaH_2PO_4, 12H_2O$ | 3,62 g |
| $H_2O$, qsp | 100 ml. |

Les viscosités en fonction des quantités de tampon sont les suivantes :

| Volume de tampon présent dans la formule | 0 | 0,25 ml | 0,5 ml | 1 ml | 2 ml | 4 ml | 6 ml | 8 ml | 10 ml |
|---|---|---|---|---|---|---|---|---|---|
| Viscosité (mPa.s) | 100 | 53 | 44 | 35 | 29 | 26 | 21 | 18 | 16 |

## Revendications

**1.** Collyre prêt à l'emploi destiné notamment au traitement de l'oeil sec comprenant en solution aqueuse :

- un polymère gélifiant polyanionique
- un tampon en une quantité suffisante pour réduire la viscosité du collyre à une valeur de 10 à 60 mPa.s (mesurée en utilisant un viscosimètre à mobile tournant HAAKE VT500 avec des cylindres coaxiaux NV comprenant un cylindre interne de rayons 17,85-20,1 mm et de hauteur 60 mm et un cylindre externe de rayons 17,5-20,5 mm et de largeur 0,35 mm à 1200 $s^{-1}$ à une température de 22°C $\pm$ 1°C) et
- une polyvinylpyrrolidone en une quantité suffisante pour que le collyre soit limpide.

**2.** Collyre selon la revendication 1, dans lequel le polymère gélifiant est un polymère de l'acide acrylique réticulé.

3. Collyre selon la revendication 2, dans lequel le polymère de l'acide acrylique réticulé est un Carbopol®.

4. Collyre selon la revendication 2 ou 3, dans lequel le polymère de l'acide acrylique réticulé est présent à une concentration de 0,1 à 1% en poids.

5. Collyre selon l'une quelconque des revendications 1 à 4, dans lequel les tampons sont choisis parmi le tampon phosphate potassique et le tampon phosphate sodique.

6. Collyre selon l'une quelconque des revendications 1 à 5, dans lequel le polyvinylpyrrolidone est la polyvinylpyrrolidone K30.

7. Collyre selon l'une quelconque des revendications 1 à 6, comprenant 0,2% en poids de polymère anionique et 1% en poids de polyvinylpyrrolidone.

8. Collyre selon l'une quelconque des revendications 1 à 7, comprenant en solution aqueuse :

0,2% de Carbopol® 980,
1% de polyvinylpyrrolidone K30,
du tampon phosphate sodique en quantité suffisante pour avoir une viscosité de 10 à 30 mPa.s (mesurée en utilisant un viscosimètre à mobile tournant HAAKE VT500 avec des cylindres coaxiaux NV comprenant un cylindre interne de rayons 17,85-20,1 mm et de hauteur 60 mm et un cylindre externe de rayons 17,5-20,5 mm et de largeur 0,35 mm à 1200 s$^{-1}$ à une température de 22°C ± 1°C).

9. Procédé pour rendre limpide une composition opalescente contenant un polymère gélifiant polyanionique et un tampon en une quantité suffisante pour réduire la viscosité de la composition à une valeur de 10 à 60 mPa.s (mesurée en utilisant un viscosimètre à mobile tournant HAAKE VT500 avec des cylindres coaxiaux NV comprenant un cylindre interne de rayons 17,85-20,1 mm et de hauteur 60 mm et un cylindre externe de rayons 17,5-20,5 mm et de largeur 0,35 mm à 1200 s$^{-1}$ à une température de 22°C ± 1°C), consistant à ajouter une quantité suffisante de polyvinylpyrrolidone pour rendre limpide la composition.

**Patentansprüche**

1. Gebrauchsfertige Augentropfen, die insbesondere zur Behandlung von trockenen Augen bestimmt sind und in wässriger Lösung umfassen:

- ein polyanionisches gelbildendes Polymer
- einen Puffer in ausreichender Menge, um die Viskosität der Augentropfen auf einen Wert von 10 bis 60 mPa·s (gemessen unter Verwendung eines HAAKE VT500-Rotationsviskosimeters mit koaxialen Zylindern NV, umfassend einen inneren Zylinder mit einem Radius von 17,85 bis 20,1 mm und einer Höhe von 60 mm und einen äußeren Zylinder mit einem Radius von 17,5 bis 20,5 mm und einer Breite von 35 mm, bei 1200 s$^{-1}$ und einer Temperatur von 22°C ± 1°C) zu verringern, und
- ein Polyvinylpyrrolidon in ausreichender Menge, dass die Augentropfen klar sind.

2. Augentropfen nach Anspruch 1, wobei das gelbildende Polymer ein vernetztes Acrylsäurepolymer ist.

3. Augentropfen nach Anspruch 2, wobei das vernetzte Acrylsäurepolymer ein Carbopcl® ist.

4. Augentropfen nach Anspruch 2 oder 3, wobei das vernetzte Acrylsäurepolymer in einer Konzentration von 0,1 bis 1 Gew.% vorliegt.

5. Augentropfen nach einem der Ansprüche 1 bis 4, wobei der Puffer aus Kaliumphosphatpuffer und Natriumphosphatpuffer ausgewählt ist.

6. Augentropfen nach einem der Ansprüche 1 bis 5, wobei es sich bei dem Polyvinylpyrrolidon um Polyvinylpyrrolidon K30 handelt.

7. Augentropfen nach einem der Ansprüche 1 bis 6, umfassend 0,2 Gew.% anionisches Polymer und 1 Gew.% Polyvinylpyrrolidon.

8. Augentropfen nach einem der Ansprüche 1 bis 7, umfassend in wässriger Lösung:

   - 0,2% Carbopol® 980,
   - 1% Polyvinylpyrrolidon K30,
   - Natriumphosphatpuffer in ausreichender Menge, um eine Viskosität von 10 bis 30 mPa·s (gemessen unter verwendung eines HAAKE VT500-Rotationsviskosimeters mit koaxialen Zylindern Nv, umfassend einen inneren Zylinder mit einem Radius von 17,85 bis 20,1 mm und einer Höhe von 60 mm und einen äußeren Zylinder mit einem Radius von 17,5 bis 20,5 mm und einer Breite von 35 mm, bei 1200 s$^{-1}$ und einer Temperatur von 22°C $\pm$ 1°C) zu erhalten.

9. Verfahren zum Klären einer trüben Zusammensetzung, die ein gelbildendes polyanionisches Polymer und einen Puffer in ausreichender Menge umfasst, um die Viskosität der Zusammensetzung auf einen Wert von 10 bis 60 mPa·s (gemessen unter Verwendung eines HAAKE VT500-Rotationsviskosimeters mit koaxialen Zylindern NV, umfassend einen inneren Zylinder mit einem Radius von 17,85 bis 20,1 mm und einer Höhe von 60 mm und einen äußeren Zylinder mit einem Radius von 17,5 bis 20,5 mm und einer Breite von 35 mm, bei 1200 s$^{-1}$ und einer Temperatur von 22°C $\pm$ 1°C) zu verringern, umfassend das Hinzufügen einer ausreichenden Menge Polyvinylpyrrolidon, dass die Zusammensetzung klar wird.

**Claims**

1. A ready-to-use eye lotion intended in particular for the treatment of dry eye condition, comprising the following in an aqueous solution:

   - a polyanionic jellifying polymer
   - a buffer in sufficient quantity to reduce the viscosity of the eye lotion to a figure of 10 to 60 mPa.s (measured using a HAAKE VT500 rotating viscosimeter with NV coaxial cylinders comprising an inner cylinder with radii 17.85 - 20.1 mm and 60 mm high and an outer cylinder with radii 17.5 - 20.5 mm and 0.35 mm wide at 1200 s$^{-1}$ at a temperature of 22°C $\pm$ 1°C) and
   - a polyvinyl pyrrolidone in sufficient quantity for the eye lotion to be clear.

2. An eye lotion according to Claim 1 in which the jellifying polymer is a polymer of the reticulated acrylic acid.

3. An eye lotion according to Claim 2 in which the polymer of the reticulated acrylic acid is a Carbopol®.

4. An eye lotion according to Claim 2 or 3 in which the polymer of the reticulated acrylic acid is present in a concentration of 0.1 to 1% by weight.

5. An eye lotion according to any of the Claims 1 to 4 in which the buffers are selected from among the potassium phosphate buffer and the sodium phosphate buffer.

6. An eye lotion according to any of the Claims 1 to 5 in which the polyvinyl pyrrolidone is polyvinyl pyrrolidone K30.

7. An eye lotion according to any of the Claims 1 to 6, comprising 0.2% by weight of anionic polymer and 1% by weight of polyvinyl pyrrolidone.

8. An eye lotion according to any of the Claims 1 to 7, comprising the following in aqueous solution:

   0.2% of Carbopol® 980
   1% of polyvinyl pyrrolidone K30
   sodium phosphate buffer in sufficient quantity to have a viscosity of 10 to 30 mPa.s (measured using a HAAKE VT500 rotating viscosimeter with NV coaxial cylinders comprising an inner cylinder of radii 17.85 - 20.1 mm and 60 mm high and an outer cylinder of radii 17.5 - 20.5 mm and 0.35 mm wide at 1200 s$^{-1}$ at a temperature of 22°C $\pm$ 1°C).

9. A process to clarify an opalescent compound containing a polyanionic jellifying polymer and a sufficient quantity of buffer to reduce the viscosity of the compound to a figure of 10 to 60 mPa.s (measured using a HAAKE VT500 rotating viscosimeter with NV coaxial cylinders comprising an inner cylinder of radii 17.85 - 20.1 mm and 60 mm high and an outer cylinder of radii 17.5 - 20.5 mm and 0.35 mm wide at 1200 s$^{-1}$ at a temperature of 22°C $\pm$ 1°C), consisting in adding a sufficient quantity of polyvinyl pyrrolidone to make the compound clear.